(19) Europäisches Patentamt
European Patent Office
Office européen des brevets

(11) **EP 4 125 814 B1**

(12) **EUROPEAN PATENT SPECIFICATION**

(45) Date of publication and mention
of the grant of the patent:
**24.09.2025 Bulletin 2025/39**

(21) Application number: **21719765.6**

(22) Date of filing: **23.03.2021**

(51) International Patent Classification (IPC):
*A61K 9/00* (2006.01)          *A61K 33/18* (2006.01)
*A61K 45/06* (2006.01)         *A61P 31/04* (2006.01)
*A61P 31/06* (2006.01)         *A61P 31/10* (2006.01)
*A61P 31/12* (2006.01)         *A61P 31/14* (2006.01)
*A61K 31/4706* (2006.01)       *A61K 31/593* (2006.01)
*A61K 31/7048* (2006.01)       *A61M 16/00* (2006.01)
*A61M 16/10* (2006.01)         *A61M 16/14* (2006.01)

(52) Cooperative Patent Classification (CPC):
(C-Sets available)
**A61K 45/06; A61K 9/007; A61K 31/4706;
A61K 31/593; A61K 31/7048; A61K 33/18;
A61M 16/024; A61M 16/109; A61M 16/14;
A61P 31/04; A61P 31/06; A61P 31/10; A61P 31/12;
A61P 31/14;** A61M 11/042;                    (Cont.)

(86) International application number:
**PCT/US2021/023574**

(87) International publication number:
**WO 2021/195017 (30.09.2021 Gazette 2021/39)**

(54) **IODINE-RELEASING COMPOUNDS FOR TREATING A RESPIRATORY CORONAVIRUS INFECTION**

IOD FREISETZENDE VERBINDUNGEN ZUR BEHANDLUNG EINER RESPIRATORISCHEN CORONAVIRUSINFEKTION

COMPOSES LIBÉRANT DE L'IODE POUR TRAITER UNE INFECTION PAR CORONAVIRUS RESPIRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **23.03.2020   US 202062993085 P
17.05.2020   US 202063026122 P
07.07.2020   IL 27590920**

(43) Date of publication of application:
**08.02.2023   Bulletin 2023/06**

(73) Proprietor: **Iocure, Inc.
Lawrence, New York 11559 (US)**

(72) Inventor: **FARB, Mark Daniel
New York 11559 (US)**

(74) Representative: **Modiano, Gabriella Diana et al
Modiano & Partners SA
Steinsdorfstraße 14
80538 München (DE)**

(56) References cited:
**WO-A1-2019/009630      WO-A2-2008/005194
US-A- 4 113 857           US-A- 5 038 769**

• **CORNELIA WIEGAND ET AL: "pH Influence on
Antibacterial Efficacy of Common Antiseptic
Substances", SKIN PHARMACOLOGY AND
PHYSIOLOGY, vol. 28, no. 3, 1 January 2015
(2015-01-01), CH, pages 147 - 158, XP055650564,
ISSN: 1660-5527, DOI: 10.1159/000367632**

**(Cont. next page)**

- **PERCIVAL STEVEN L. ET AL: "Antiseptics for treating infected wounds: Efficacy on biofilms and effect of pH", CRITICAL REVIEWS IN MICROBIOLOGY, vol. 42, no. 2, 26 August 2014 (2014-08-26), GB, pages 293 - 309, XP055814526, ISSN: 1040-841X, DOI: 10.3109/ 1040841X.2014.940495**
- **ASÍ LOVE ENCARNA!: "COVID-19 Desinfección vahos y gárgaras anti virus y bacterias del aparato respiratorio", YOUTUBE, 19 April 2020 (2020-04-19), pages 1 pp., XP054981911, Retrieved from the Internet <URL:https://www. youtube.com/watch?v=X8_Dgh6k6GI&t=55s> [retrieved on 20210616]**
- **EGGERS MAREN: "Infectious Disease Management and Control with Povidone Iodine", INFECTIOUS DISEASES AND THERAPY, vol. 8, no. 4, 14 August 2019 (2019-08-14), pages 581 - 593, XP055814238, ISSN: 2193-8229, DOI: 10.1007/s40121-019-00260-x**

(52) Cooperative Patent Classification (CPC): (Cont.)
A61M 16/1095; A61M 16/202; A61M 2016/1035;
A61M 2202/0468; A61M 2202/203; A61M 2202/206;
A61M 2205/18; A61M 2205/3324; A61M 2205/3331;
A61M 2205/502

C-Sets
**A61K 31/4706, A61K 2300/00;
A61K 31/593, A61K 2300/00;
A61K 31/7048, A61K 2300/00;
A61K 33/18, A61K 2300/00**

## Description

### FIELD OF THE INVENTION

Disclosed herein are methods for treating respiratory infections, comprising use of a heated iodine salt solution.

### BACKGROUND

There are many respiratory viruses, the most recent serious one being current coronavirus (COVID-19). Influenza is widespread and killed 60,000 people in the US in one recent winter. There are other hard to treat pathogens, such as SARS, respiratory syncytial virus, resistant tuberculosis, antibiotic resistant pneumonias, candidiasis, and more.

Viruses are a large problem because the antibiotics for them are rare and not very effective.

There are respiratory bacterial pathogens that do not respond well to antibiotics. As one example, the treatment of tuberculosis is meeting increasing drug resistance.

In summary, there is a need for a broadly effective antibiotic useful against many kinds of respiratory system pathogens, particularly viruses.

### IODINE AND IODINE COMPOUNDS IN MEDICINE

Iodine solution is a known antiseptic for skin, for aqueous solutions, and for air sterilization.

WO2019/009630 discloses a solid composition comprising an iodine agent and sodium chloride having improved water solubility and to an antiviral and antimicrobial composition for an eye, oral cavity, nasal cavity or inhalation containing an aqueous solution thereof, wherein the solid composition comprises an iodine agent and sodium chloride or a mixture of an iodine agent, sodium chloride and a flavoring agent and is excellent in not only storage stability but also water solubility as a dissolution rate thereof for water is 30 seconds or less.

WO2008/005194 discloses method for killing or substantially eradicating a pathogen in the upper respiratory tract of a mammal comprising generating molecular iodine in situ using an oxidant-reductant reaction with a minimum concentration of at least about 25 ppm of I2 and I2 comprises at least 40% of the total iodine atom.

US4113857 discloses an improved process for preparing iodophor preparations which comprises adding an ion selected from the group consisting of iodate, bromate, chlorite, chromate, hypochlorite, permanganate and persulfate ions and hydrogen peroxide to the iodine carrier selected from the group consisting of povidone, cationic, anionic and nonionic detergent compounds and an iodide salt, to result in a substantially pure iodophor compound which is virtually free of autodegraded iodide ion contamination and unreacted elemental iodine, said iodophor compounds exhibiting an enhanced stability.

US5038769 discloses a method and apparatus for the delivery of a vaporized pharmaceutical or medicant for the treatment and alleviation of ailments and/or for the delivery of medicants.

Percival Steven L.. et al.,: "Antiseptics for treating infected woulds: Efficacy on biofilms and effect of pH" (Critical Reviews in Microbiology, vol 42, no.2, 26 August 2014, pages 293-309) reviews the effectiveness of various antiseptics, particularly silver and iodine-based compounds, in combating biofilms within chronic wounds.

https://www.youtube.com/watch?v=X8 Dgh6k6GI&t=55s was published on April 19, 2020.

Cornelia Wiegand et al.,: "pH influence on Antibacterial Efficacy of Common Antiseptic Substances" (Skin Pharmacology and Physiology, vol 28, no.3, 1 January 2015, pages 147-158) examines how pH levels affect the antimicrobial effectiveness of antiseptics like povidone-iodine, silver nitrate, chlorhexidine, octenidine and polyhexanide against *Staphylococcus aureus* and *Pseudomonas aeruginosa.*

Iodine compounds have other medical uses. Potassium iodine has long been used for thyroid conditions and to block radioactive iodine uptake in nuclear emergencies.

The overwhelming consensus of doctors is that iodine is unsafe for other uses. Almost every doctor has seen in standard textbooks such as Harrison's Principles of Medicine and Goodman and Gilman's pharmacology texts that iodine is toxic above a certain level for inspiration, and there is an additional perception that iodine is unsafe for mucosal surfaces, as death can occur from excessive gastrointestinal exposure to iodine. PubChem (https://pubchem.nchi.nlm.nib.gov/compound/Iodine# section=NIPH-Clinical-Trials-Search-of-Japan) only lists an antibiotic use of iodine on the skin.

There are also commercial gargles using iodine (Eggers et al. 2018, In Vitro Bactericidal and Virucidal Efficacy of Povidone-Iodine Gargle/Mouthwash Against Respiratory and Oral Tract Pathogens. Infect Dis Ther. 2018 Jun; 7(2): 249-259) and potassium iodide has been used as lavage fluid (Derscheid et al., Am J Respir Cell Mol Biol. 2014 Feb; 50(2): 389-397. *Increased Concentration of Iodide in Airway Secretions Is Associated with Reduced Respiratory Syncytial Virus Disease Severity.*)- all at room temperature.

Scientific American's June 2020 issue featured COVID. An article (Waldholz) on fasttrack drugs does not even consider the use of iodine or any treatment other than complex antiviral drugs and various immunological treatments. That reflects state of the art thinking about how to deal with severe viral respiratory infections.

When the applicant wrote an email to a pulmonologist at a major medical center about using iodine for his COVID patients, he responded with an article downloaded from Fisher Scientific about the toxicity of iodine. It contains the phrase "Harmful if inhaled"!

## SUMMARY OF THE INVENTION

The present invention is defined by the appended claims. Any embodiments which do not fall under the scope of the claims are not the subject of the present invention.

The references to the method of treatment of this description are to be interpreted as references to compounds, pharmaceutical compositions and medicaments of the present invention for use in a method of treatment of the human (or animal) body by a therapy.

The present invention regards a composition, comprising a vapor, for use in treating a respiratory infection by administration to a respiratory tract of a subject infected with a viral respiratory pathogen which is coronavirus, wherein said vapor is generated by warming an aqueous solution comprising an iodine-releasing ionic compound which is potassium iodide or povidone iodine and elemental iodine , thereby generating said vapor. Here is a summary of the inventive steps that will be discussed in more detail. The applicant is leveraging the properties of iodine chemistry in conjunction with information from diverse and unconnected medical specialties and environmental biology to innovate a way of treating difficult respiratory infections.

    a. The antibiotic agent is in the form of an aqueous vapor. The applicant has found no reference in the literature of using aqueous iodine vapor to treat infections.

    b. The vapor is heated. Since heating a substance is generally regarded to increase its activity and toxicity, there is no reference in the literature to using a heated aqueous vapor of iodine in treatment.

    c. The solution forming the vapor contains not only iodine but also an iodine-releasing salt. ("Salt" by definition includes substances such as Povidone which are organic but ionize in water.) Some compounds containing dissolved iodine release it into solution. This is the first time that the nature of such compounds to form equilibrium mixtures in water has been leveraged to deliver active elemental iodine, which is what reacts with pathogens, in a much safer form. The small percentage of elemental iodine reacts with the pathogen in the respiratory tract, and immediately more is drawn out of the solution to restore equilibrium.

    d. In an embodiment, the upper temperature is limited (typically to 80 deg. C) so that not too much elemental iodine is released. Below a certain temperature, there is minimal activity. This is a safety precaution based on applying equilibrium and heated solution effects to the current problem.

    e. The parts per million can be increased beyond the limits thought possible, because those limits were based on pure iodine vapor, whereas, in solution accompanied by a salt, generally at least 90% of the iodine is dissolved in salt form.

    f. The dosage per day can be capped below toxic levels, and the total allowed amount per day can be concentrated into a smaller amount of time that is more reasonable for treating a patient in a potentially critical situation because the chemistry allows the use of a higher concentration. (The possibilities for toxicity are both from the concentration of free elemental iodine at any one time and the total amount allowed per day; the new method of delivery enables a solution.)

    g. Iodine is more effective against pathogens at a higher pH. That alkalinity can be delivered as part of the aqueous vapor and was not relevant when pure elemental iodine vapor was used. The alkalinity can also be used to reduce the dose and thereby the toxicity.

The applicant challenges the perception that iodine is unsafe on mucosal surfaces from some little-known ophthalmic research on the successful and safe use of substances such as Povidone iodine drops for conjunctival sterilization prior to eye surgery. (One reference is Isenberg and Apt. The Ocular Application of Povidone-Iodine, Community Eye Health. 2003; 16(46): 30-31.) In addition, the applicant, an ophthalmologist, occasionally prescribed Povidone drops for patients with methicillin-resistant Staphylococcus Aureus eye infections; he received calls in all cases from nurses, pharmacists, or doctors like "Dr. Farb, do you know what you are doing?" After they received literature references and proceeded, the applicant had a 100% success rate in treating those infections. Since most pulmonologists and infectious disease experts do not read the ophthalmic literature, they would find such an idea dangerous, as typified by the referenced email.

The next question is how to deal with iodine's known respiratory toxicity.

The applicant disputes the prevailing medical practice by using another discipline, physical chemistry of the halogens, which virtually all doctors are unaware of. Iodine-releasing ionic compounds such as Povidone and potassium iodide (as opposed to substances containing iodine such as thyroxine wherein the iodine is bound to other atoms and not capable of being released into salt form without breaking the bonds) exist in an equilibrium in a solution with elemental (also termed molecular) iodine. Here is the key formula for an example of potassium iodide, a likely source of the iodine treatment and the ingredient in Lugol's Solution:

$$KI(aq) + I_2(s) \rightarrow KI_3(aq)$$

$I_3$ is highly soluble, therefore only small amounts of $I_2$ are available while in solution. That reduces the toxicity from inspiration. When the $I_2$ reacts with pathogens, the equilibrium draws out the creation of more $I_2$ from $KI_3$. Because the iodine-releasing compounds are highly soluble, there is little free iodine present in these solutions, maybe 1-10% according to various studies.

Another example is hypoiodous acid, HIO. It rapidly decomposes: $5\ HIO \rightarrow HIO_3 + 2\ I_2 + 2\ H_2O$.

Since free iodine is what reacts with and inactivates pathogens, and most body cells can handle it (iodine in solution is required for basic health and circulates in the blood to be used in the thyroid gland), providing iodine in heated and inspired solution to the respiratory tract would be a way to apply it so it would have little toxic effect until it lands on a surface and attacks pathogens.

Warming such an iodide salt solution would have additional benefits of making it easier to reach the lungs and anywhere else in the respiratory system, and of making it more reactive for the first few seconds until it reaches body temperature.

Steaming an aqueous solution of iodine-releasing compound that forms an equilibrium small amount of free iodine, as far as the applicant knows, has never been considered before, likely because of the difficulty bridging the disciplines of infectious disease and pulmonary medicine, ophthalmology, pharmacology, and physical chemistry, and the simultaneous fear of iodine being painful, toxic, and messy.

Without wishing to be bound by theory, iodine in association with warm, moist air elicits a more vigorous reaction from the higher temperature and loosening of any layers of material, whether the product of human or pathogenic metabolism, deposited on the lung surface, thereby enabling greater therapeutic efficacy. It has been reported that COVID-19 emits a coating on the lung's mucosal surface. This method of treatment would help to penetrate that layer.

DOSAGE AND TOXICITY

An alternative is the use of higher than supposedly safe doses because short pulses at high parts per millions can kill the virus in emergency situations, and the published toxicity standards are made for prolonged exposure. If one were to use the traditional way of looking at iodine, with dosage according to number of iodine molecules or weight of iodine in the solution, the treatment proposed here would appear to be more toxic than it really is. The reason is that so little elemental iodine is present-and even that is partially retained in solution-that there is little toxicity in the passage through the respiratory tract until it lands on the infected surfaces.

For comparison, the recommended dose of iodine for hyperthyroidism is 750 mg/day. Recommended dose to prevent iodine uptake in the presence of radioactive iodine: 130 mg/day for an adult (CDC). Normal nutrition is 0.150 mg/day. According to Poisoning & Drug Overdose, 6e, 2012, Kent R. Olson, page 1499, short term exposure can be irritating at as low as 0.1 ppm and work is difficult at 1.5-2 ppm. The ACGIH-recommended workplace ceiling limit (TLV-C) for iodine vapor is 0.1 ppm (1 mg/m3). The air level considered immediately dangerous to life or health (IDLH) is 2 ppm. (Owen, Kelly, Poisoning and Drug Overdose, Access Medicine [McGraw Hill Medical], Chapter 84, Iodine, by Kelly P. Owen ) The Burnet and Stone research suggests complete inactivation of viruses within 1 minute in vitro at 0.1 ppm. Decreased virulence of viruses occurred at 0.005 ppm.

The CDC website (https://www.cdc.gov/nceh/radiation/emergencies/ki.htm) states as follows: "According to the FDA, the following doses are appropriate to take after internal contamination with (or likely internal contamination with) radioactive iodine: Newborns from birth to 1 month of age should be given 16 mg (¼ of a 65 mg tablet or ¼ mL of solution). This dose is for both nursing and non-nursing newborn infants. Infants and children between 1 month and 3 years of age should take 32 mg (½ of a 65 mg tablet OR ½ mL of solution). This dose is for both nursing and non-nursing infants and children. Children between 3 and 18 years of age should take 65 mg (one 65 mg tablet OR 1 mL of solution). Children who are adult size (greater than or equal to 150 pounds), i.e. 68,04 kg) should take the full adult dose, regardless of their age. Adults should take 130 mg (one 130 mg tablet OR two 65 mg tablets OR two mL of solution). Women who are breastfeeding should take the adult dose of 130 mg."

A toxicity study for the EU (https://echa.europa.eu/registration-dossier/-/registered-dossier/5883/7/6/2) showed data for an experiment on rats ingesting liquid potassium iodide for a 2-year period. A short bottom line is that 10 ppm showed no long-term effects, and only 100 ppm showed a long-term decrease in life span.

In some embodiments, the concentration of iodine will be lower in vapor than in solution. In certain embodiments, e.g., to avoid toxicity, the resulting air mixture iodine concentration in the lungs or respirator input tubes is less than 2 ppm for extended-period dosing. In other embodiments, this is calculated based on, e.g., the equilibrium concentration of iodine in the solution and the temperature and the flow rate of the input air. In yet other embodiments, pulses of higher than 2 ppm respirator air iodine are used, since Bennett and Stone found virus kill rates superior at higher concentrations than 2 ppm. In this manner, kill rate can be increased by the temporary use of a higher dose. Such a higher dose may be, in some embodiments, above a daily toxicity level if it were continuously administered for an entire day.

WHO Guidelines for Drinking-water Quality, 1996, 2nd ed. Vol. 2, states as follows: "Doses of 30-250 ml of tincture of iodine (about 16-130 mg of total iodine per kg of body weight) have been reported to be fatal. Acute oral toxicity is primarily due to irritation of the gastrointestinal tract, marked fluid loss and shock occurring in severe cases."

Yeon and Jung (Yeon and Jung, Effects of temperature and solution composition on evaporation of iodine as a part of estimating volatility of iodine under gamma irradiation, Nuclear Engineering and Technology, Volume 49, Issue 8, December 2017, Pages 1689-1695.) performed $I_2$ evaporation experiments with $I_2$ and I-mixed solutions in the temperature range 26-80°C in an open, well-ventilated space. The evaporation of $I_2$ was observed to follow primarily first order kinetics, depending on the $I_2$ concentration. The evaporation rate constant increased rapidly with increase in temperature. Their figure 4 shows that evaporation at 50 degrees Centigrade is over 10 times more rapid than at 26 degrees, room temperature, and over 30 times at 80 degrees.

In light of the above information, and the fact that iodine vapor readily penetrates the vascular system from the lungs, it is reasonable to be cautious and take the lowest of the above toxicity measures into account as a maximum, that is, 16 mg/kg/day. For a margin of safety, we suggest that the total iodine content be no greater than 8 or 10 mg/kg/day. As a result, the combination of not exceeding 2 ppm in vapor (for other than short pulses) maximum and 16 mg/kg/day would keep the patient from the lowest levels of generally recognized toxicity, and that in practice making the maximums 10 mg/kg/day and 0.1 ppm would clearly be safe. Even with respirator use, it is likely that planning on a 1 ppm level would mean that much would be lost through the air, so there is a built-in safety factor for both the lung dose and the dose absorbed into the blood. Therefore, the applied dosage is likely to be effective at 10% or less of the potentially toxic dose.

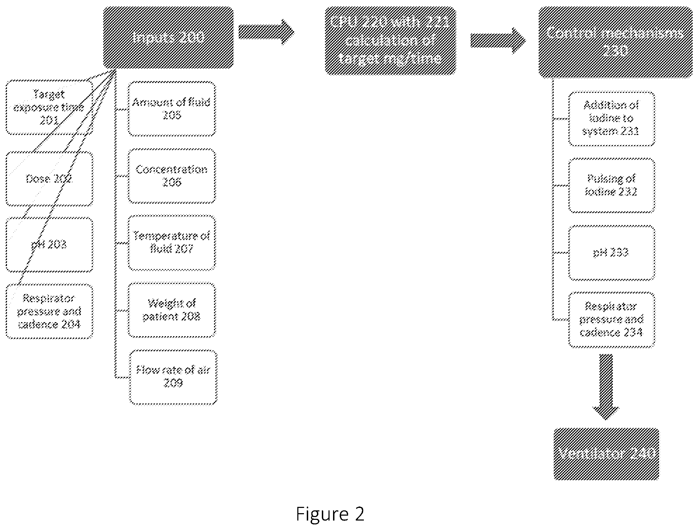

Let us take a case with Lugol's iodine. Using 0.3 ml of Lugol's solution per minute at 50 degrees and a delivery rate of 6 liters of air per minute (the average), a solution concentration of 0.1 ppm will result in 248 minutes of treatment in order to deliver the maximum non-toxic dose per day for an average adult. To make this more realistic, raising the concentration to 1 ppm and increasing cadence and quantity of breaths to 12 liters of air per minute results in a treatment time of 12 minutes to reach the maximum non-toxic dose for the day, If the doctor were to decide that a patient could take up to 250 mg of iodide per day, and if that were to be delivered over a short period of time at 1 ppm, it would take several minutes to deliver. In certain embodiments, to enable levels of iodine that are safe but have anti-pathogenic activity, the ventilator or other iodine delivery device (which may be any device described herein) calculates the total number of milligrams per kilogram (mg/kg) per day - based on the volume of air introduced to the patient and the concentration of the iodine in total and/or in bioactive form - such that the total never goes above 16 mg/kg per day, and that it can be set to lower and safer levels such as (in various embodiments) 15, 10, 8, 5, or 1 mg/kg per day, with the ability to customize, in some embodiments, the total amount administered for patients with conditions such as thyroid disease. The ventilator or other iodine delivery device can also be set to make a time-based calculation, so that the patient would, for example, receive 15 mg/kg or less in the course of one hour, once per day, to enable a more concentrated regimen of iodine with a higher number of ppm than would be possible if the patient received a maintenance dose steadily throughout 24 hours. There is a basis for that in the Bennett and Stone *in vitro* studies of a greater viral kill rate from higher concentrations. This is summarized in Figure 2.

IN VITRO

The literature on the effectiveness of iodine on viruses in a solution is almost entirely on cold iodine solutions and is in all cases outside the human body, for the purpose of sanitizing the air or water.

As reported in Letters in Applied Microbiology (51(2):158-63), Andreas Sauerbrei and Peter Wutzler studied successful use within 5 minutes of iodine against a variety of viruses on the skin.

In 1945, Stone and Burnet (Stone and Burnet, The Action Of Halogens On Influenza Virus With Special Reference To The Action Of Iodine Vapour On Virus Mists, Australian Journal of Experimental Biology and Medical Science, 1 Sept. 1945; https://onlinelibrary.wiley.com/doi/abs/10.1038/icb.1945.32) created a bin in which a virus mist was exposed to iodine vapor. The experiment involved the placement of iodine crystals in those bins to produce the vapor. Another way they produced the vapor involved dissolving iodine crystals in methanol. They found that 0.1 parts per million destroyed the influenza viruses. Below that, there was a destructive effect but not total. This was the effect of a mist on a mist, not an *in vivo* infection. In all cases, they introduced the iodine in the test chamber before introducing the mice (not a typical therapeutic scenario). They were interested in air sterilization. They give the practical suggestion to impregnate gauze masks for doctors and nurses with iodine. They published a related article as Burnet *et al.* (Burnet et al., Action of iodine vapour on influenza virus in droplet suspension, Aust J Sci. 1945 Feb;7:125) This approach was not tested in humans and was used in prevention and antisepsis with non-infected mice, instead of treatment. Iodine was neither vaporized with steam nor nebulized.

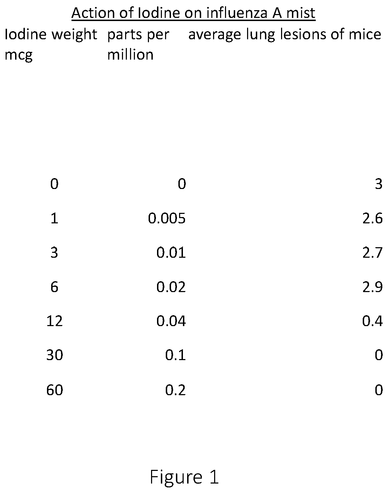

**Figure 1** (not encompassed by the present invention) is a table from Stone *et al.* on the effective concentrations for virus kills in the outside air (not in the lungs). The inventor proposes that the concentration of iodine in the inspired air should ideally be in the range of 0.01-0.2 ppm based on this data, but it should be safe to go to 2 ppm, in certain embodiments. However, the chemistry of iodine solutions would enable higher ppm of iodine in air droplets. The inventor suggests that short pulses of relatively higher concentrations of iodine (*e.g.,* 0.1 ppm and higher) would have a faster therapeutic effect without risking toxicity rather than a lower concentration for a longer time. This can be, in certain embodiments, adjusted by a control mechanism, in some embodiments computerized, coupled with a timer in the ventilator or other iodine delivery device.

Eggers 2019, Infectious Disease Management and Control with Povidone Iodine, Maren, Infectious Diseases and Therapy volume 8, pages 581-593(2019)) states: "Following application, elemental iodine can take on several forms in aqueous solution, with the molecular $I_2$ and hypoiodous acid (HOI) being the most effective in terms of antimicrobial activity. The iodine molecules are free to oxidise vital pathogen structures such as amino acids, nucleic acids and membrane components. An equilibrium is achieved in such circumstances, with more PVP-bound iodine released into solution to replace the iodine that is consumed by germicidal activity. The maintenance of this equilibrium ensures long-lasting efficacy during bouts of microorganism proliferation, as well as better tolerability for patients due to lower levels of irritation. Electron microscopy and biochemical observations support the hypothesis that PVP-I disrupts microbial cell walls by inducing pore formation, leading to cytosol leakage. The lack of reported resistance to PVP-I to date is thought to be due to the sheer diversity of susceptible targets within each pathogen, an important aspect to be considered in the face of rising concerns for antibiotic resistance." They continue, "PVP-1 refers to an iodine preparation commonly used in both household and healthcare settings. It consists of a complex of povidone, hydrogen iodide, and elemental iodine which targets structures critical to the survival and replication of microorganisms. Common formulations typically consist of a 10% PVP-I solution containing 1 % available iodine."

The above studies illustrate how the amount of potentially toxic iodine is limited in solution and is safer than previous medical practice allows.

CASE STUDY

The inventor suffered from a coronavirus infection and cured himself with inhaled iodine vapor, as detailed in the following diary entries:
On the evening of Monday, March 16, I came back to my Long Island apartment from a day in Manhattan passing through Penn Station (after a previous week of flying from Tel Aviv to Amsterdam, then Amsterdam to JFK, and taking trains within the Netherlands). In the Netherlands I sat next to, for an extended time, a co-worker who became very sick a few days later. Feeling OK.

Tuesday: March 17: Started to get what I thought was a cold.

Wed: March 18-Friday March 20: I started to realize that I didn't just have a cold, as this wasn't following my usual cold symptoms. I started to get fevers and sweats in spite of taking Tylenol every 4 hours until Sunday morning March 22, a huge degree of exhaustion (needing to go back to sleep after being up around 30-60 minutes), a moderate cough, moderate shortness of breath (for me, that meant doing a low intensity 20-minute workout instead of a high intensity 1-2 hour daily workout, and feeling exhausted and short of breath). I also noticed that my ability to taste food had decreased.

Friday, March 20: I realized I had corona, and started to think through my medical experience and knowledge about a cure.

Sunday, March 22, 8:30 AM: I was sweating with fever and could barely make it to my car. I brought with me a vaporizer with water, a lighter to AC electrical converter, and put on a mask. I went to the local pharmacy to buy liquid iodine (both tincture and Povidone). I parked my car, put iodine in the vaporizer (around one fluid ounce, i.e. 29.57 ml, in around a quart, i.e. 946.35 ml, of water), and breathed from the vaporizer with the windows mostly shut for an hour. I took a break, refilled the vaporizer, and repeated the process. By 12:00, I was feeling 95% better, walking around my apartment and working, just needed a small nap, No fever, no need for Tylenol. I've been doing fine since. I did supplement with some 5-minute breathing sessions over a pot with some water and some iodine after that but didn't really need it.

Several weeks later I had a positive antibody test for corona and two negative nasal swab tests for corona.

pH

In still other embodiments, pH is adjusted to improve effectiveness of iodine. Hsu and Nomura (Hsu and Nomura, Sterilization Action Of Chlorine And Iodine On Bacteria And Viruses In Water Systems (US Army Technical Report)) found in Experiment 10 that higher pH resulted in a higher kill rate. In certain embodiments, substances that can lend a higher pH to the iodine/vapor combination, without it becoming caustic to the lungs, constitute a method and formulation to enhance the treatment's effectiveness. Gomez *et al.,* proposed that inhalation of aerosol of a bicarbonate solution, resulting in a higher pH between 7 and 8 in most cases, reduced sputum viscosity in cystic fibrosis. (Gomez et al., Safety, Tolerability, and Effects of Sodium Bicarbonate Inhalation in Cystic Fibrosis. Clinical Drug Investigation, Nov. 13, 2019). Another formulation could include carbonate or hydroxide.

Eschenbacher WL, Gross KB, Muench SP, Chan TL (Eschenbacher WL, Gross KB, Muench SP, Chan TL, Am Rev Respir Dis. 1991 Feb;143(2):341-5. *Inhalation of an alkaline aerosol by subjects with mild asthma does not result in bronchoconstriction)* (https://www.ncbi.nlm.nih.gov/pubmed/1990950) found that asthma patients had no reactive vasoconstriction with alkaline vapor as high as pH 10.3. This means that an alkaline solution or vapor with iodine up to pH 10.3 would be safe and likely more effective than iodine alone.

DEFINITIONS

The respiratory system consists of the mouth, nose, sinuses, pharynx (upper respiratory system); and trachea and lungs (lower respiratory system). Most respiratory pathogens affect both, but the infection in the lungs is usually more serious. Infections in the trachea and/or lungs are referred to herein as a lower respiratory infection.

Influenzas (not encompassed by the present invention) and especially coronaviruses affect or penetrate the mucosal lining of the lung in their most serious form. Given the inventor's belief in the safety and efficacy of iodine against coronaviruses on mucus membranes, an additional question is how to get it into the lungs.

A warmed solution is defined as a solution above 25 degrees °C (room temperature), which results, in certain embodiments, in release of therapeutically effective amounts of volatile iodine *(e.g.,* at 26 °C or higher) - or, in other embodiments, within another temperature range mentioned herein, each of which represents a separate embodiment. Realistically, heating to at least 30 degrees substantially improves the delivery and reactivity.

## BRIEF DESCRIPTION OF THE DRAWINGS

Fig. 1 (not encompassed by the present invention) is a table from Stone *et al.* on the effective iodine concentrations for kills of Influenza in a mist.

Fig. 2 (not encompassed by the present invention) is a schematic depiction of exemplary, non-limiting inputs 200 for calculating a dose of inhaled iodine, including, for example, target exposure time 201, dose 202, pH 203, respirator cadence and/or pressure 204, fluid amount 205, concentration 206, fluid temperature 207, patient weight 208, and air flow rate 209. Inputs are used to program CPU 220 and which in turn calculates target milligrams of iodine inspired per time 221 and instructs control mechanisms 230, including, for example, addition of iodine to system 231 *(e.g.,* via addition of iodine or an iodine-releasing compound to solution in the device, or via supplying additional iodine-containing solution to the device), iodine pulsing 233, pH 233, and respirator cadence and/or pressure 234. This figure is not meant to limit the number of inputs. As discussed herein, the expected volume of the patient's respiration could be a factor used in calculating the ppm over a period of time. It is possible that the computer may be programmed with a default calculation of the patient's breath volume based on age, weight, or height, or it may rely on the volume of the actions of the respirator or other factors.

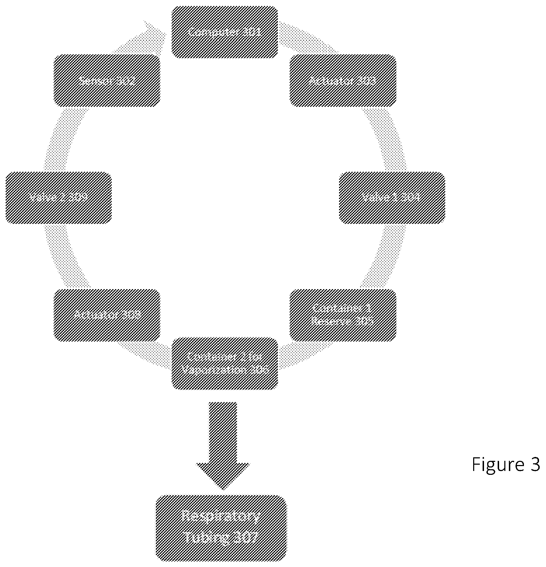

Fig. 3 (not encompassed by the present invention) is a schematic of an exemplary iodine, alkali, or other formulation autofill process in conjunction with a respirator. A computer 301 with memory receiving input from at least one sensor 302 instructs a first actuator 303 to open a first valve 304 to release therapeutic material (not shown) from a reserve container 305 to a vaporization container (306), from which there is a connection via respiratory tubing 307 to the patient's respiratory system (not shown). That container is also connected via control by computer 301 to a downstream actuator 308 and second valve (309) in order to open or block the communication to the respiratory system. This computer control relies on at least one sensor 302, which may be a volume sensor, whereby the volume of therapeutic substance or equivalent measure is taken in order to determine proper dosing and proper movement through the first valve 304. In the case of alkali autofill, or a combination of alkali and Iodine, for example, there may be an optional pH sensor. The most important is to sense volume so it is clear when the vaporization container 305 needs to be refilled.

## DETAILED DESCRIPTION

Provided herein is a composition, comprising a vapor, for use in treating a respiratory infection by administration to a respiratory tract of a subject infected with a viral respiratory pathogen which is coronavirus, wherein said vapor is generated by warming an aqueous solution comprising an iodine-releasing ionic compound which is potassium iodide or povidone iodine and elemental iodine.

In some embodiments, there is provided a composition for use in treating a respiratory infection by administration to a respiratory tract of a subject infected with a viral respiratory pathogen, which is coronavirus, comprising a vapor, for administration to a respiratory tract of a subject infected with a respiratory pathogen, wherein: (a) the vapor is generated by warming an aqueous solution having a pH of 7.0-10.0, the solution comprising an iodine-releasing ionic compound which is potassium iodide or povidone iodine and elemental iodine in a total iodine amount of 1.5-10 ppm, to a temperature of 30-80 degrees centigrade (°C), thereby generating the vapor; (b); the administration is for 2 hours or less per day; and (c) total iodine delivered to the patient's respiratory tract does not exceed 8 mg/kg of body weight/day.

In various embodiments, there is provided a composition comprising a vaporcomprising elemental iodine and an iodine-releasing compound which is potassium iodide or povidone iodine, for use in treating a respiratory infection by administration to a lower respiratory tract of a subject infected with coronavirus

In other embodiments, there is provided a composition for use in treating a subject infected with a lower respiratory pathogen, which is coronavirus, said use comprising administering to the subject's lower respiratory tract a vapor comprising elemental iodine and an iodine-releasing compound which is potassium iodide or povidone iodine.

The use of saturated potassium iodide solution (commercially available as SSKI®) is one standard in use and is described herein. One embodiment as an example would involve diluting 1 ml of SSKI by a factor of 10. Then 1 ml would contain 76.4 mg of iodine, which is well within the safe dosage for a day. That 1 ml could be vaporized to the patient's lungs over a sequence of time chosen by the doctor. Then, in another embodiment, for an example only, that amount could be timed for delivery so that the parts per million in the volume of inspired air would be 0.1 ppm, Since reports of discomfort according to NIOSH start at 1.5 ppm, it should be safe for a limited time to set the concentration to 1 ppm in order to have a stronger virus kill that is well within the limits of safety. In more extreme cases of danger to the patient from the respiratory infection, the doctor could decide to increase the concentration, for an example, up to 1.9 or other target ppm on a time-limited basis such as 10 minutes. The vapor is indicated for administration to a lower respiratory tract of a subject infected with a respiratory pathogen, which is coronavirus. According to the invention, the solution is warmed subsequent to introduction of the iodine and iodine-releasing compound which is potassium iodide or povidone iodine into the solution.

In some embodiments, the solution is disposed in a ventilator. In other embodiments, the solution is disposed within an evaporation device operably connected with a ventilator, e.g., so that the vapor produced by the solution is introduced into the ventilator.

In other embodiments, the solution is disposed in an outpatient device, which may be, in certain embodiments, a non-invasive breathing assistance apparatus, e.g., a breathing tube or respiratory face mask. In other embodiments, the outpatient device is a room humidifier, household pot, or any other type of evaporation-facilitating device that does not require a professional to operate.

Iodine, iodine-releasing ionic compounds, and solutions comprising same

The term "iodine" is used herein to refer to the element itself, e.g., in its common molecular form "I subscript 2". Iodine-releasing compounds are potassium iodide or povidone iodine . Solutions containing iodine and iodine-releasing compounds are encompassed in the described compositions. According to the invention, the described compounds are able to generate bioavailable iodine (I sub 2) for the purpose of vaporization. In certain embodiments, iodine is present in the described vapor in a therapeutically effective amount, that is, an amount having antimicrobial activity. In other embodiments, iodine is present in any of the amounts or ranges mentioned herein.

The described iodine-releasing compounds which are potassium iodide or povidone iodine (of any composition for use in treating a respiratory infection by administration to a respiratory tract of a subject infected with a viral respiratory pathogen, which is coronavirus mentioned herein) include compounds that release free elemental iodine. The term is not intended to encompass compounds that do not release free elemental iodine or another active iodine compound in solution or vapor, even if they comprise iodine atoms - non-limiting examples of such compounds are raw seaweed and thyroid hormone.

In certain embodiments, the iodine-releasing compound for vaporization has a boiling point below 200 °C (or, in other embodiments, below 150 °C, 125 °C, or 100 °C); or, in other embodiments, a room-temperature half-life in aqueous solution of at least 30 minutes (or, in other embodiments, at least 45 minutes, 60 minutes, 90 minutes, or 120 minutes); or, in other embodiments, both characteristics, which may be freely combined.

In other embodiments, the described iodine-releasing compound (of any composition mentioned herein) is povidone iodine (2-Pyrrolidinone, 1-ethenyl-homopolymer), or potassium iodide.

It will be appreciated by those skilled in the art, in light of the present disclosure, that the term "active iodine compound(s)" refers to iodine-containing compounds with significant antiviral activity at concentrations achievable by the described devices. In other embodiments, e.g., in the case of vaporized compositions, additional advantageous characteristics are sufficient lability to be vaporized at effective concentrations and sufficient stability to survive the journey from the device to the subject's respiratory tract.

Solely by way of exemplification, 0.1 ppm of iodine = 1.038 mg/m^3. Potassium iodide can be conveniently prepared as a saturated solution, abbreviated SSKI. SSKI contains about 764 mg iodide per mL. There are around 15 drops per mL; the iodide dose is therefore approximately 51 mg per drop. Therefore, to achieve 0.1 ppm in a cubic meter of air, put one drop of SSKI in 50 drops of water.

The uncomplexed molecular iodine (I sub 2) is, the active ingredient in the described iodine solution. This is the amount that could be used for dosage calculation, and for the method of determining the amount of biocidal uncomplexed iodine in ventilators, combined with the understanding that a new equilibrium is continuously reached. The concentration of other compounds containing iodine can also be measured, but the dosage calculation based on I sub 2 is used, in certain embodiments, for greater ease of standardization. Such calculations are known to those skilled in the art; non-limiting examples of them are provided in Wadai *et al.* (Wada et al., Relationship between Virucidal Efficacy and Free Iodine: Concentration of Povidone-Iodine in Buffer Solution, Biocontrol Science, 2016, Vol. 21, No. 1, 21 -270).

In yet other embodiments, the described solution (of any composition for use in treating a respiratory infection by administration to a respiratory tract of a subject infected with a viral respiratory pathogen, which is coronavirus mentioned herein) comprises both elemental iodine and an iodine-releasing ionic compound which is potassium iodide or povidone iodine . As provided herein, the presence of an iodine salt increases the solubility of elemental iodine in aqueous solutions.

Further, the presence of both elemental iodine and an potassium iodide ( in equilibrium) enables replenishing of elemental iodine levels, enabling maintenance of elemental iodine levels within a therapeutic range over the course of many hours.

According to the invention, as the iodine solution is heated and molecular iodine andthe iodine-releasing ionic compound which is potassium iodide or povidone iodine evaporate with the steam, the molecular iodine in the solution is replenished by reaching a new equilibrium with the salts or carriers still in solution.

The iodine-containing solution of the described composition contains, in some embodiments, molecular iodine at a solution concentration of 0.1-10 parts per million (ppm); or, in other embodiments, 0.2-10 ppm, 0.3-10 ppm, 0.4-10 ppm, 0.5-10 ppm, 0.6-20 ppm, 0.8-20 ppm, 1-20 ppm, 0.2-15 ppm, 0.3-15 ppm, 0.4-15 ppm, 0.5-15 ppm, 0.6-15 ppm, 0.8-15 ppm, 1-15 ppm, 0.2-10 ppm, 0.3-10 ppm, 0.4-10 ppm, 0.5-10 ppm, 0.6-10 ppm, 0.8-10 ppm, 1-10 ppm, 1-10 ppm, 2-10 ppm, 3-10 ppm, 4-10 ppm, or 5-10 ppm. In certain embodiments, the dosage is calibrated such that the iodine content in the vapor administered in one day is less than 8 mg/kg of body weight of the subject.

The iodine-containing vapor of the described composition contains, in some embodiments, molecular iodine at a <u>vapor</u> concentration of 0.01-2 parts per million (ppm); or, in other embodiments, 0.02-2 ppm, 0.03-2 ppm, 0.04-2 ppm, 0.05-2 ppm, 0.06-2 ppm, 0.08-2 ppm, 0.1-2 ppm, 0.02-1.5 ppm, 0.03-1.5 ppm, 0.04-1.5 ppm, 0.05-1.5 ppm, 0.06-1.5 ppm, 0.08-1.5 ppm, 0.1-1.5 ppm, 0.02-1 ppm, 0.03-1 ppm, 0.04-1 ppm, 0.05-1 ppm, 0.06-1 ppm, 0.08-1 ppm, 0.1-1 ppm, 0.1.5-1 ppm, 0.2-1 ppm, 0.3-1 ppm, 0.4-1 ppm, or 0.5-1 ppm. In certain embodiments, the dosage is calibrated such that the iodine content in the vapor administered in one day is less than 8 mg/kg of body weight of the subject.

In yet other embodiments, e.g., for an exposure time of 24 hours or less, molecular iodine is present in the described vapor at a concentration over 1.5 ppm, over 2 ppm, over 3 ppm, over 4 ppm, or over 5 ppm. In other embodiments, iodine is present in the solution at a concentration over 15 ppm, over 20 ppm, over 30 ppm, over 40 ppm, or over 50 ppm. The exposure time of the subject to the vapor is, in various embodiments, less than 20 hours, less than 16 hours, less than 12 hours, less than 10 hours, less than 8 hours, less than 6 hours, less than 4 hours, less than 3 hours, or less than 2 hours. The aforementioned iodine concentrations and exposure times may be freely combined. In still other embodiments, a vapor containing very high concentrations of iodine, *e.g.,* 2-10 ppm, 3-10 ppm, 4-10 ppm, 5-10 ppm, 2-5 ppm, or 2-4 ppm is administered to a subject for 30-60 minutes; for 15-30 minutes; or for 1-15 minutes. In certain embodiments, the treatment may be administered up to twice daily.

In other embodiments, e.g., for a subject with thyroid disease, molecular iodine is present in the solution at a concentration of 0.1-5 ppm; or, in other embodiments, 0.2-5 ppm, 0.3-5 ppm, 0.4-5 ppm, 0.5-5 ppm, 0.6-5 ppm, 0.8-5 ppm, or 1-5 ppm.

In certain embodiments, the described iodine-containing vapor is produced by warming a solution or mixture comprising iodine (and/or, in various embodiments, an iodine-containing compound) to a temperature above 25 degrees centigrade (°C). In more specific embodiments, the solution or mixture is warmed to 26-80 °C, 30-80 °C, 35-80 °C, 40-80 °C, 45-80 °C, 50-80 °C, 55-80 °C, 60-80 °C, 65-80 °C, 70-80 °C, 75-80 °C, 80-80 °C, 26-100 °C, 30-100 °C, 35-100 °C, 40-100 °C, 45-100 °C, 50-100 °C, 55-100 °C, 60-100 °C, 65-100 °C, 70-100 °C, 75-100 °C, or 80-100 °C.

In certain embodiments, the described vapor has been obtained from a solution that has a pH between 7.0-10.3, between 7.0-10.0, between 7.0-9.5, between 7.0-9.0, between 7.5-10.3, between 7.5-10.0, between 7.5-9.5, or between 7.5-9.0.

In other embodiments, the described vapor, when allowed to condense, has a pH between 7.0-10.3, between 7.0-10.0, between 7.0-9.5, between 7.0-9.0, between 7.5-10.3, between 7.5-10.0, between 7.5-9.5, or between 7.5-9.0.

<u>Iodine delivery systems</u>

According to the present disclosure, all systems of providing air (including oxygen), vapor, or medication to a subject's respiratory system can be employed as means of delivering the claimed composition for use in treating a respiratory infection by administration to a respiratory tract of a subject infected with a viral respiratory pathogen, which is coronavirus. That includes for example ventilators, humidifiers, vaporizers, and both specialized and non-specialized heating containers *(e.g.,* a kitchen pot) that can produce warmed or steaming vapor. The terms "ventilator" is intended to encompass any type of apparatus that physically contains and influences the composition of the air inspired by a subject. In certain embodiments, the described ventilator is closed, or, in other embodiments, at least partially closed. In other embodiments, the ventilator comprises a breathing tube. Enclosed respirator systems, e.g., PAPR (powered air-purifying respirators), are included. An Ambu bag (Bag valve mask) is considered another embodiment of a ventilator. "Face masks" refer to masks enabling respiration of inspired air, with or without medication, and are also considered, in another embodiment, a component of ventilators (protective face masks are not encompassed) Nasal prongs for air inhalation, usually supplementary oxygen, are also, in another embodiment, a component of ventilators. In certain embodiments, the described ventilator is a mechanical ventilator, defined as a mechanized device that enables the delivery or movement of air and/or oxygen into the lungs of a patient whose breathing has ceased, is failing, or is inadequate. In further embodiments, the mechanized ventilator has any of the following attributes (alone or in combination): a. monitors and customizes gas delivery; b. maintains a minimal pressure in the lungs (e.g., to prevent the alveoli from collapsing), and c. delivers air and/or oxygen to the lungs by way of a tube inserted into the trachea through the mouth or nose.

The inventor proposes that the treatment will be better in those ventilators that use heated wires to maintain water vapor saturation and warm temperatures, because there will be less water vapor condensation (and thus iodine condensation in the tubes or upper respiratory system), thus enabling the iodine to reach the lungs. In the same way, if a patient is breathing from a vaporizer or a pot of heated water with iodine added, he should inhale close to the source to make sure more iodine reaches the lungs. Deep breaths should be encouraged in all cases, and particularly coronavirus, which causes a coating on the lung surface, so that peripheral areas of the lung dependent on mucociliary clearance obtain a greater benefit from the treatment. The inventor's recommendation is to make sure the ventilator is set to maximize iodine exposure to the lung periphery. Since it is important that the iodine reaches the periphery of the lungs, the option of adjusting the pressure and periods of inhalation (cadence) to reach the lung periphery should be available as part of the control system advocated by the applicant.

Due to some reports (Swift, Diana, Medscape News, April 13, 2020, Higher Mortality Rate in Ventilated COVID-19 Patients in Large Sample (Medscape, article 928605) that excessive pressure from respirators may increase morbidity from coronavirus, it is suggested that, in some embodiments, such use of increased pressure be used only for short time periods coinciding with increased dosing of the iodine.

In some embodiments, the ventilator, humidifier, vaporizer, or heating container of the described composition comprises an iodine autofill system. Alternatively or in addition, the ventilator or other iodine delivery device comprises an alkali autofill system, which, in other embodiments, is configured to maintain a target pH range of the solution contained therein, which may be, in various embodiments, any pH or pH range mentioned herein. In certain embodiments, that alkali can be bicarbonate or a bicarbonate salt.

In certain embodiments, the iodine autofill system helps maintain exposure to the lungs over an extended period. The extent can be set by an autofill controller and an apparatus comprising a computer control system with memory that releases a set amount of compound, held in a container communicating with the ventilator and equipped with a valve controlled by the computer, during a particular time range. This can be combined, in certain embodiments, with a sensor to detect the concentration of iodine, and the sensor sends data to the computer, which then sends instructions to the valve controller. In other embodiments, a pH sensor is included to monitor the pH of the ventilator fluid and have an autofill operating in a similar fashion for an alkali such as sodium bicarbonate to maintain the pH at a particular number. The computer can be set to compile data of the amount dosed over time in order to keep the total amount dosed below toxicity level and provide it via a user interface, by wireless or cable communication with the computer, and to generate alerts locally and via the computer. An input device or interface can be attached to the autofill to customize the regimen for a patient's weight and other conditions.

The described vapor is, in various embodiments, disposed within a breathing tube, a respiratory face mask, or nasal prongs, each of which represents a separate embodiment. In still other embodiments, the vapor is disposed within a ventilator. In yet other embodiments, the vapor is disposed within a humidifier or vaporizer.

Alternatively or in addition, the ventilator, humidifier, vaporizer, or heating container comprises a heating element (non-limiting examples of which are heated wire(s) or other immersed element, a heated plate [which is, in some embodiments, adjacent to the chamber holding the solution], and an element surrounding the chamber holding the solution) that facilitates vaporization of water.

Target Pathogens

The pathogen treated by any of the mentioned compositions is coronavirus.In other embodiments, the coronavirus described herein is human and bat severe acute respiratory syndrome coronavirus (SARS-CoV) of the type severe acute respiratory syndrome-related coronavirus, e.g. SARS-CoV-1 and SARS-CoV-2. In certain embodiments, the treated virus is SARS-CoV-2.

Eggers 2019 states "The influenza virus has been responsible for some of the most significant epidemics in the modern world, with annual outbreaks resulting in approximately 3-5 million cases of severe illness and between 250,000 and 500,000 deaths per year. An influenza study using plaque inhibition assays showed that a 1.56-mg/ml PVP-I treatment can inhibit infections in MDCK cells by human (eight strains) and avian (five strains) influenza A viruses, including H1N1, H3N2, H5N3 and H9N2, from 23 to 98%. Receptor binding analysis revealed that haemagglutinin inhibition was the likely cause of the PVP-I virucidal activity, rather than the inhibition of host-specific sialic acid receptors. The finding also demonstrates two specific mechanisms of reduction of viral growth, namely, PVP-I blockade of viral attachment to the host cell receptors and the inhibition of viral release from infected cells."

Alternatively or in addition, some target pathogen express neuraminidase. Eggers 2019 states "PVP-I formulations are also known to have broad antiviral properties. These effects are mechanistically similar in principle to iodine's antibacterial activity. For example, the virucidal mechanisms of action of PVP-I have been determined to involve the inhibition of essential viral enzymes such as neuraminidase. The inactivation of this enzyme blocks viral release from the host cell, preventing further spread of the virus to uninfected cells. In addition, PVP-I also inhibits viral haemagglutinin, resulting in the blockade of attachment to host cell receptors. By simultaneously targeting both critical aspects of the viral machinery needed for replication, PVP-I reduces the likelihood of resistance emerging through sudden mutation."

Possible mechanisms of action

In certain embodiments, without wishing to be limited by theory, the described compositions for use in treating a respiratory infection by administration to a respiratory tract of a subject infected with a viral respiratory pathogen which is coronavirus exert an effect by modification of surface proteins and/or fatty acids. McDonnell and Russell write, "Similar to chlorine, the antimicrobial action of iodine is rapid, even at low concentrations, but the exact mode of action is unknown. Iodine rapidly penetrates into microorganisms and attacks key groups of proteins (in particular the free-sulfur amino acids cysteine and methionine), nucleotides, and fatty acids, which culminates in cell death. Less is known about the antiviral action of iodine, but nonlipid viruses and parvoviruses are less sensitive than lipid enveloped viruses. Similar to bacteria, it is likely that iodine attacks the surface proteins of enveloped viruses, but they may also destabilize membrane fatty acids by reacting with unsaturated carbon bonds."

Additional agents

In another embodiment, heparin or other anti-clotting agent is co-administered. One value of the use of iodine, particularly in patients with slow blood clotting times, is to minimize the use of anti-clotting medications in this complication of corona.

Currently, there is much concern about coronavirus causing blood clots, so it is of interest that the iodine reduces hemagglutination *in vitro.* Sriwilaijaroen (Sriwilaijaroen et al., Mechanisms of the action of povidone-iodine against human and avian influenza A viruses: its effects on hemagglutination and sialidase activities, Virology Journal volume 6, Article number: 124 (2009)) states: "Receptor binding inhibition and hemagglutinin inhibition assays indicated that PVP-I affected viral hemagglutinin rather than host-specific sialic acid receptors." According to the research cited, the agglutination is related to the virus more than the host.

In certain embodiments, ACE inhibitors (Rohan), vitamin D (McCall), ivermectin, corticosteroids, of which one example is dexamethasone (Giardina), mouthwash (Vlessides)-particularly those with high content of germicidal compound(s) such as alcohol, and/or steroids or other anti-viral compounds are co-administered, each of which represents a separate embodiment.

In certain embodiments, the additional compound is administered in the same composition as the iodine and iodine-releasing compound.

Administration with drugs targeting intracellular pH or lysosomal pH

In yet other embodiments, the described, composition for use in treating a respiratory infection by administration to a respiratory tract of a subject infected with a viral respiratory pathogen, which is coronavirus, further comprises or utilizes an additional active agent that increases intracellular pH or lysosomal pH. In some, the drug is chloroquine or hydroxychloroquine. Other non-limiting examples of active agents that increases intracellular pH or lysosomal pH are aminoquinolines, for example 4-aminoquinolines, such as amodiaquine, hydroxychloroquine (HCQ), chloroquine; 8-aminoquinolines, such as primaquine and pamaquine; and mefloquine. In certain embodiments, the additional active agent is administered in the same composition as the iodine or iodine-releasing compound.

The inventor suggests combining the iodine treatment with other drugs such as chloroquine, which may inhibit the pathogens in complementary ways, for example, by increasing intracellular pH or pH of endosomes or lysosomes of target cells of the pathogen in the lower respiratory tract. Krogstad and Schlesinger (Am J Trop Med Hyg. 1987 Mar;36(2):213-20, *The basis of antimalarial action: non-weak base effects of chloroquine on acid vesicle pH.*) write, "Biologically active concentrations of chloroquine increase the pH of the parasite's acid vesicles within 3-5 min." Alternatively or in addition, a pH-raising agent is included in an iodine solution. The present disclosure encompasses each of these embodiments as a new drug combination, whatever the route of administration.

In yet other embodiments, iodine solution with added pH-raising agent is used concurrently with oral chloroquine treatment.

Subjects

In certain embodiments, the compositions for use in treating a respiratory infection by administration to a respiratory tract of a subject infected with a viral respiratory pathogen, which is coronavirus, is for use in treating a human. In other embodiments, is for use in treating an animal, non-limiting examples of which are dogs, cats, horses, and cows.

**Claims**

1. A composition, comprising a vapor, for use in treating a respiratory infection by administration to a respiratory tract of a subject infected with a viral respiratory pathogen, which is coronavirus, wherein:

(a) said vapor is generated by warming an aqueous solution comprising an iodine-releasing ionic compound, which is potassium iodide or povidone iodine, and elemental iodine, thereby generating said vapor.

## Patentansprüche

1. Eine Zusammensetzung, die einen Dampf umfasst, zur Verwendung in der Behandlung einer Atemwegsinfektion durch Verabreichung in einen Atemwegstrakt eines Individuums, das mit einem viralen Atemwegs-Pathogen, welches Coronavirus ist, infiziert ist; wobei

   (a) der Dampf erzeugt wird durch Erwärmung einer wässerigen Lösung, die Folgendes umfasst: eine Iod freisetzende ionische Verbindung, welche Kaliumiodid oder Povidon-Iod ist, und elementares Iod, wodurch der Dampf erzeugt wird.

## Revendications

1. Composition comprenant une vapeur, destinée à être utilisée dans le traitement d'une infection respiratoire par administration dans les voies respiratoires d'un sujet infecté par un agent pathogène respiratoire viral, qui est le coronavirus, où :

   (a) ladite vapeur est générée par chauffage d'une solution aqueuse comprenant un composé ionique libérant de l'iode, qui est l'iodure de potassium ou la povidone iodée, et de l'iode élémentaire, générant ainsi ladite vapeur.

## Action of Iodine on influenza A mist

| Iodine weight mcg | parts per million | average lung lesions of mice |
|---|---|---|
| 0 | 0 | 3 |
| 1 | 0.005 | 2.6 |
| 3 | 0.01 | 2.7 |
| 6 | 0.02 | 2.9 |
| 12 | 0.04 | 0.4 |
| 30 | 0.1 | 0 |
| 60 | 0.2 | 0 |

Figure 1

Figure 2

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019009630 A
- WO 2008005194 A
- US 4113857 A
- US 5038769 A

### Non-patent literature cited in the description

- **PERCIVAL STEVEN L. et al.** Antiseptics for treating infected woulds: Efficacy on biofilms and effect of pH. *Critical Reviews in Microbiology*, 26 August 2014, vol. 42 (2), 293-309
- **CORNELIA WIEGAND et al.** pH influence on Antibacterial Efficacy of Common Antiseptic Substances. *Skin Pharmacology and Physiology*, 01 January 2015, vol. 28 (3), 147-158
- **EGGERS et al.** Bactericidal and Virucidal Efficacy of Povidone-Iodine Gargle/Mouthwash Against Respiratory and Oral Tract Pathogens. *Infect Dis Ther.*, June 2018, vol. 7 (2), 249-259
- **DERSCHEID et al.** *Am J Respir Cell Mol Biol.*, February 2014, vol. 50 (2), 389-397
- **ISENBERG** ; **APT**. The Ocular Application of Povidone-Iodine. *Community Eye Health.*, 2003, vol. 16 (46), 30-31
- **KENT R. OLSON**. *Poisoning & Drug Overdose*, 2012, vol. 6e, 1499
- Iodine. **KELLY P. OWEN**. Poisoning and Drug Overdose, Access Medicine. McGraw Hill Medical
- *WHO Guidelines for Drinking-water Quality*, 1996, vol. 2
- **YEON** ; **JUNG**. Effects of temperature and solution composition on evaporation of iodine as a part of estimating volatility of iodine under gamma irradiation. *Nuclear Engineering and Technology*, December 2017, vol. 49 (8), 1689-1695
- **ANDREAS SAUERBREI** ; **PETER WUTZLER**. *Letters in Applied Microbiology*, vol. 51 (2), 158-63
- **STONE** ; **BURNET**. The Action Of Halogens On Influenza Virus With Special Reference To The Action Of Iodine Vapour On Virus Mists. *Australian Journal of Experimental Biology and Medical Science*, 01 September 1945, https://onlinelibrary.wiley.com/-doi/abs/10.1038/icb.1945.32
- **BURNET et al.** Action of iodine vapour on influenza virus in droplet suspension. *Aust J Sci.*, February 1945, vol. 7, 125
- **EGGERS** ; **MAREN**. Infectious Disease Management and Control with Povidone Iodine. *Infectious Diseases and Therapy*, 2019, vol. 8, 581-593
- **GOMEZ et al.** Safety, Tolerability, and Effects of Sodium Bicarbonate Inhalation in Cystic Fibrosis. *Clinical Drug Investigation*, 13 November 2019
- **ESCHENBACHER WL** ; **GROSS KB** ; **MUENCH SP** ; **CHAN TL**. *Am Rev Respir Dis.*, February 1991, vol. 143 (2), 341-5
- **WADA et al.** Relationship between Virucidal Efficacy and Free Iodine: Concentration of Povidone-Iodine in Buffer Solution. *Biocontrol Science*, 2016, vol. 21 (1), 21-270
- **SWIFT, DIANA**. Higher Mortality Rate in Ventilated COVID-19 Patients in Large Sample. *Medscape News*, 13 April 2020
- **SRIWILAIJAROEN et al.** Mechanisms of the action of povidone-iodine against human and avian influenza A viruses: its effects on hemagglutination and sialidase activities. *Virology Journal*, 2009, vol. 6
- **KROGSTAD** ; **SCHLESINGER**. *Am J Trop Med Hyg.*, March 1987, vol. 36 (2), 213-20